Europäisches Patentamt

European Patent Office

Office européen des brevets

(19)

(11) Veröffentlichungsnummer: **0 281 662 B1**

# (12) EUROPÄISCHE PATENTSCHRIFT

(45) Veröffentlichungstag der Patentschrift: **16.10.91**

(51) Int. Cl.⁵: **A61F 6/04**

(21) Anmeldenummer: **87104401.2**

(22) Anmeldetag: **25.03.87**

(54) **Element zur Aufnahme eines Kondoms.**

(30) Priorität: **27.02.87 DE 3706313**

(43) Veröffentlichungstag der Anmeldung:
**14.09.88 Patentblatt 88/37**

(45) Bekanntmachung des Hinweises auf die
Patenterteilung:
**16.10.91 Patentblatt 91/42**

(84) Benannte Vertragsstaaten:
**AT BE CH DE ES FR GB GR IT LI LU NL SE**

(56) Entgegenhaltungen:
**DE-A- 3 237 566**
**US-A- 2 567 926**

(73) Patentinhaber: **Hulik, Jan**
**Beim Herbstenhof 38**
**W-7400 Tübingen(DE)**

Patentinhaber: **Martin, Claus**
**Lichtensteiner Strasse 22**
**W-7408 Wankheim(DE)**

(72) Erfinder: **Hulik, Jan**
**Beim Herbstenhof 38**
**W-7400 Tübingen(DE)**
Erfinder: **Martin, Claus**
**Lichtensteiner Strasse 22**
**W-7408 Wankheim(DE)**

(74) Vertreter: **Ott, Elmar, Dipl.-Ing.**
**Kappelstrasse 8**
**W-7240 Horb 1(DE)**

## Beschreibung

Die Erfindung bezieht sich auf ein Element zur Aufnahme eines Kondoms.

Die zur Empfängnisverhütung und zum Schutz gegen ansteckende Krankheiten dienenden Kondome werden üblicherweise im aufgerollten Zustand in dünnwandigen Plastikhüllen verpackt. Eine solche Plashkhülle ist aus DE-A-3237566 bekannt Beim Aufreißen dieser Plastikhüllen besteht die Gefahr der Beschädigung des Kondoms. Außerdem ist die Handhabung der Kondome umständlich, da stets darauf geachtet werden muß, daß der Kondom sich nur in einer Richtung abrollen läßt.

Der Erfindung liegt die Aufgabe zugrunde, ein Element zur Aufnahme eines aufgerollten Kondoms zu schaffen, welches die Handhabung des Kondoms erleichtert.

Die Lösung dieser Aufgabe wird durch die im Hauptanspruch angegebenen Merkmale erhalten. Der aufgerollte Kondom ist in einem ringförmigen Hohlprofil sicher gelagert, wobei das vordere Ende des Kondoms aus einem Ringspalt herausragt. Zur Benutzung des Kondoms ist es somit nur noch erforderlich, das ringförmig ausgebildete Element mit dem Kondom über den Penis zu streifen, wobei sich der Wickel des Kondoms im Hohlprofil abrollt. Ist der Kondom vollständig abgerollt, kann das ringförmige Element in entgegengesetzter Richtung vom Penis abgenommen werden.

Das ringförmig ausgebildete Element besteht vorzugsweise aus einem hinreichend stabilen Kunststoff, der den eingelegten Wickel vor Beschädigungen schützend umschließt. Der Wickel des Kondoms ist mit einer Vorspannung im erfindungsgemäßen Element gelagert, die dadurch erzielt wird, daß der Innendurchmesser des Hohlprofils größer ist als der Innendurchmesser des Wickels. Durch diese aufgrund geringfügiger Aufweitung entstehende mechanische Spannung wird ein problemloses Abwickeln des Wickels bei der Anwendung gewährleistet.

Das Element ist vorzugsweise als Ring mit C-förmigem Hohlprofil ausgebildet, wobei der offene Spalt kleiner als der Wickeldurchmesser ausgebildet sein kann.

Im Bereich des Ringspaltes kann auch ein radial verlaufender Vorsprung angeformt sein, der den Wickel gegen unbeabsichtigtes Herausrutschen vor dem vollständigen Abrollen sichert.

Die Erfindung wird nachfolgend anhand der Zeichnung näher erläutert. Es zeigen:

Figur 1 ein ringförmiges Element mit im Hohlprofil eingesetzten Kondom im Schnitt,

Figur 2 die Ansicht der Ringfläche mit Ringspalt des in Figur 1 dargestellten Elements und

Figur 3 ein weiteres Ausführungsbeispiel für das Hohlprofil.

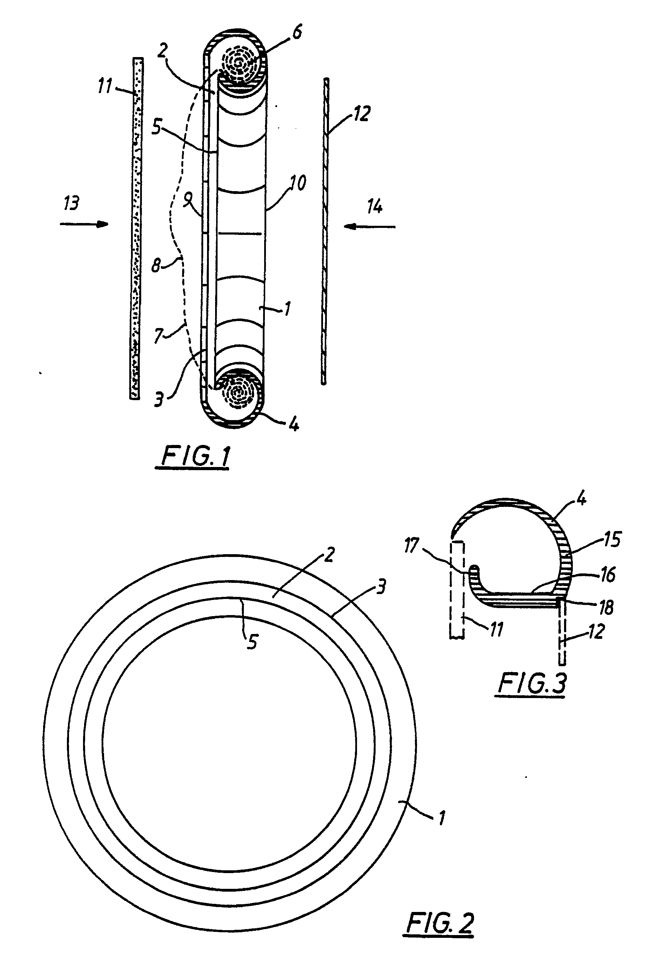

Bei dem in Figur 1 dargestellten ringförmigen Element 1 ist ein Ringspalt 2 ausgebildet, der von einem Vorsprung 3 des Hohlprofils 4 in radialer Richtung nach außen begrenzt ist. Die andere Begrenzung des Ringspaltes 2 wird von einer gegenüber dem Vorsprung 3 zurückspringenden Kante 5 gebildet.

Das im wesentlichen C-förmig ausgebildete Hohlprofil 4 enthält den Wickel 6 eines Kondoms 7, der hier mit unterbrochenen Linien dargestellt ist. Das vordere Ende 8 des Kondoms 7 ragt aus dem Ringspalt 2 heraus, während der Wickel 6 elastisch in das Hohlprofil 4 eingespannt ist.

Die beiden Stirnseiten 9, 10 werden mit Schutzscheiben 11, 12 abgedeckt, die in Pfeilrichtungen 13, 14 auf das Element 1 aufgesetzt werden. Die Schutzscheibe 11 besteht aus einem stabileren Material, während die Schutzscheibe 12 aus einer verhältnismäßig dünnen Folie bestehen kann. Die Schutzscheiben 11, 12 schützen den Kondom 7 gegen unbeabsichtigte Beschädigung, Außerdem kann die Schutzscheibe 11 nur in Pfeilrichtung 14 vom Element 1 nach außen abgedrückt werden. Dadurch wird gewährleistet, daß das Element 1 und damit der darin gelagerte Kondom 7 nur von der Seite 10 aus über den Penis gestreift werden kann, wobei sich der Wickel 6 des Kondoms 7 abrollt. Ist der Wickel 6 vollständig abgerollt, kann das Element 1 in entgegegesetzter Richtung abgenommen werden.

Die Schutzscheibe 12 ist aus einer derart dünnen Folie, daß diese problemlos eingedrückt werden kann.

In Figur 2 ist die den Ringspalt 2 enthaltene Stirnseite des Elements 1 dargestellt.

Figur 3 zeigt ein anderes Profil 15 für das Hohlprofil 4. Das Profil 15 besitzt hier einen waagerecht verlaufenden Bereich 16, der den Wickel 6 des Kondoms 7 aufnimmt. Ein daran anschließender, radial gerichteter Rand 17 sichert den Wickel 6 gegen Herausrutschen vor dem vollständigen abwickeln. Die Schutzscheiben 11, 12 sind hier ebenfalls vorgesehen, wobei die Schutzscheibe 12 in einen entsprechenden zurückspringenden Absatz 18 am Element 1 eingreift.

Anstelle eines aufgewickelten Kondoms kann der im Element gelagerte Teil des Kondoms in das Hohlprofil eingeschoben sein, wobei eine Faltung entsteht.

Es können am Element 1 äußere Markierungen oder dergleichen vorgesehen sein, die es erlauben, die beiden Seiten des Elements 1 mittels des Tastsinnes zu unterscheiden.

## Patentansprüche

1. Element zur Aufnahme eines Kondoms,
   **dadurch gekennzeichnet,** daß das Element (1) zur Aufnahme des ringförmigen Wickels (6) oder dergl. des Kondoms (7) ein ringförmiges Hohlprofil (4) mit einer als Ringspalt (2) ausgebildeten Öffnung hat, und daß das vordere Ende (8) des eingesetzten Kondoms (7) aus dem Ringspalt (2) herausragt.

2. Element nach Anspruch 1, **dadurch gekennzeichnet**, daß der Wickel (6) des Kondoms (7) elastisch vorgespannt im Element (1) gelagert ist.

3. Element nach einem der Ansprüche 1 oder 2, **dadurch gekennzeichnet**, daß das Element als Ring mit C-förmigem Hohlprofil (4) ausgebildet ist.

4. Element nach einem der vorhergehenden Ansprüche, **dadurch gekennzeichnet**, daß ein am Hohlprofil (4) ausgebildeter Vorsprung (5, 17) oder dergleichen den Wickel (6) eines eingesetzten Kondoms (7) gegen Herausrutschen sichert.

5. Element nach einem der vorhergehenden Ansprüche, **dadurch gekennzeichnet**, daß der Ringspalt (2) schmaler ausgebildet ist als der Durchmesser des Wickels (6).

6. Element nach einem der vorhergehenden Ansprüche, **dadurch gekennzeichnet**, daß der durch das Hohlprofil (4) gebildete Ring einen Innendurchmesser von etwa 3,5 cm hat.

7. Element nach einem der vorhergehenden Ansprüche, **dadurch gekennzeichnet**, daß das Element zu beiden Seiten (9, 10) von jeweils einer Schutzscheibe (11, 12) abgedeckt ist, wobei eine der Schutzscheiben (11, 12) nur durch den freien Innenraum des ringförmigen Elements (1) nach außen wegdrückbar ist.

8. Element nach Anspruch 7, **dadurch gekennzeichnet**, daß die nach außen wegdrückbare Schutzscheibe (11, 12) aus einem stabilen Material, wie Karton, Kunststoff oder dergleichen besteht, während die andere Schutzscheibe (12) aus einem leicht zerstörbaren Material wie Papier, Kunststofffolie oder dergleichen besteht.

9. Element nach einem der vorherigen Ansprüche, **dadurch gekennzeichnet**, daß der im Hohlprofil gelagerte Teil des Kondoms (7) in das Hohlprofil (4) in Falten eingeschoben ist.

10. Element nach einem der vorhergehenden Ansprüche, **dadurch gekennzeichnet**, daß die äusere Gestaltung des Elements (1) Markierungen, Vorsprünge oder dergl. hat, die es erlauben die beiden Seiten des Elements (1) mit Hilfe des Tastsinnes zu unterscheiden.

**Claims**

1. Element for receiving a condom, characterized in that the element (1) for receiving the circular roll (6) or the like of the condom (7) has a circular hollow profile (4) with an opening constructed as an annular clearance (2) and that the front end (8) of the inserted condom (7) projects from the annular clearance (2).

2. Element according to claim 1, characterized in that the roll (6) of the condom (7) is placed in elastically pretensioned manner in the element (1).

3. Element according to one of the claims 1 or 2, characterized in that the element is constructed as a ring with a C-shaped hollow profile (4).

4. Element according to one of the preceding claims, characterized in that a projection (5, 17) or the like formed on the hollow profile (4) prevents the roll (6) of an inserted condom (7) from slipping out.

5. Element according to one of the preceding claims, characterized in that the annular clearance (2) is narrower than the diameter of the roll (6).

6. Element according to one of the preceding claims, characterized in that the ring formed by the hollow profile (4) has an internal diameter of approximately 3.5 cm.

7. Element according to one of the preceding claims, characterized in that the element is covered on the either side (9, 10) by in each case one protective disk (11, 12), one of the protective disks (11, 12) only being outwardly forcible through the free inner space of the circular element (1).

8. Element according to claim 7, characterized in that the outwardly forcible protective disk (11, 12) is made from a stable material, such as cardboard, plastic or the like, whilst the other protective disk (12) is made from a material which can easily be destroyed, such as paper, plastic film or the like.

9. Element according to one of the preceding claims, characterized in that the part of the condom (7) placed in the hollow profile (4) is inserted in the latter in folds.

10. Element according to one of the preceding claims, characterized in that the outer design of the element (1) has markings, projections, etc., which make it possible to differentiate the two sides of the element (1) with the aid of the sense of touch.

**Revendications**

1. Elément (d'emballage) pour loger un préservatif ou condom, caractérisé en ce que l'élément (1) comporte, pour loger l'enroulement (6) annulaire ou une forme analogue du préservatif (7), un profilé creux (4) annulaire comportant une ouverture en forme de fente annulaire (2), et en ce que l'extrémité avant (8) du préservatif (7) introduit fait saillie de la fente (2) annulaire.

2. Elément selon la revendication 1, caractérisé en ce que l'enroulement (6) du préservatif (7) est placé avec une précontrainte élastique dans l'élément (1).

3. Elément selon l'une des revendications 1 ou 2, caractérise en ce que l'élément a la forme d'un anneau présentant un profil (4) creux en forme de C.

4. Elément selon l'une des revendications précédentes, caractérisé en ce qu'une saillie (5, 17), formée sur le profilé creux (4) ou la partie analogue, empêche l'enroulement (6) d'un préservatif (7), qui est introduit dans le profilé, d'en sortir par glissement.

5. Elément selon l'une des revendications précédentes, caractérisé en ce que la lente annulaire (2) est plus petite que le diamètre de l'enroulement (6).

6. Elément selon l'une des revendications précédentes, caractérisé en ce que l'anneau formé par le profilé creux (4) a un diamètre interne d'environ 3,5 cm.

7. Elément selon l'une des revendications précédentes, caractérisé en ce que l'élément est recouvert des deux côtés (9, 10) chaque fois par un panneau protecteur (11, 12), l'un des panneaux protecteurs (11, 12) ne pouvant être expulsé vers l'extérieur qu'en passant par l'espace intérieur libre de l'élément (1) de forme annulaire.

8. Elément selon la revendication 7, caractérisé en ce que le panneau protecteur (11, 12), pouvant être expulsé vers l'extérieur, consiste en une matière stable, comme du carton, de la matière plastique ou une matière analogue, cependant que l'autre panneau protecteur (12) consiste en une matière facile à déchirer, comme du papier, une feuille de matière plastique ou une matière analogue.

9. Elément selon l'une des revendications précédentes, caractérisé en ce que la partie du préservatif (7) placée dans le profilé creux est introduite sous forme plissée dans le profilé creux (4).

10. Elément selon l'une des revendications précédentes, caractérisé on ce que la configuration extérieure de l'élément (1) comporte des marques, des saillies ou parties analogues permettant de différencier au toucher les deux côtés de l'élément (1).

FIG.1

FIG.2

FIG.3